# EUROPÄISCHE PATENTSCHRIFT

(11) **EP 2 836 132 B1**
(45) Veröffentlichungstag und Bekanntmachung des Hinweises auf die Patenterteilung: **03.05.2017**
(21) Anmeldenummer: 13704112.5
(22) Anmeldetag: 13.02.2013
(51) Int. Cl.: A61B 8/00

(54) **WÄRMEVORRICHTUNG**
HEATING DEVICE
DISPOSITIF POUR CHAUFFER

(30) Priorität: 12.04.2012 AT 4382012
(43) Veröffentlichungstag der Anmeldung: 18.02.2015
(73) Patentinhaber: SPS MED TEC GmbH, 4710 Grieskirchen (AT)
(72) Erfinder: SPIESSBERGER-EICHHORN,Peter, 4701 Bad Schallerbach (AT)
(74) Vertreter: KLIMENT & HENHAPEL
(86) Internationale Anmeldenummer: PCT/EP2013/052882
(87) Internationale Veröffentlichungsnummer: WO 2013/152880

(56) Entgegenhaltungen:
- CN-A- 101 718 465
- CN-Y- 201 208 265
- JP-A- 2007 195 881
- KR-B1- 100 891 873
- US-B1- 6 575 906

## Beschreibung

Die Erfindung betrifft eine Wärmevorrichtung für flaschen- oder tubenförmige Behälter gelartiger Medien zur medizinischen Diagnostik, bei dem ein beidseits geöffnetes hülsenförmiges Gehäuse mit zwei Öffnungen und einer zylindermantelförmigen, elektrisch beheizten Innenfläche vorgesehen ist, in dessen erste Öffnung der flaschen- oder tubenförmige Behälter einschiebbar ist, gemäß dem Oberbegriff von Anspruch 1.

In der medizinischen Diagnostik sind mitunter gelartige Medien auf den Körper des Patienten aufzubringen, etwa in Form eines Übertragungsgels für Ultraschalluntersuchungen oder Gleitgels für Untersuchungsinstrumente in der gynäkologischen oder endoskopischen Praxis. Diese gelartigen Medien werden in flaschen- oder tubenförmigen Behältern gelagert und werden in der Regel nicht vorgewärmt, sodass die Patienten mitunter ein unangenehmes Kälteempfinden verspüren. Es wäre daher wünschenswert, geeignete Wärmevorrichtungen für flaschen- oder tubenförmige Behälter gelartiger Medien zur Verfügung zu haben. Wärmevorrichtungen für Flüssigkeiten sind zwar etwa in Form von Flaschenwärmer aus der Säuglingspflege bekannt, bei denen ein Wasserbad elektrisch erwärmt wird, in das der flaschen- oder tubenförmige Behälter gestellt wird. Im Haushaltsbereich mögen Wärmevorrichtungen dieser Art, bei denen ein Wasserbad erwärmt wird, durchaus praktikabel sein, zur Erwärmung flaschen- oder tubenförmiger Behälter für gelartige Medien zur medizinischen Diagnostik sind sie aber aus verschiedenen Gründen ungeeignet. So kann etwa beim Entnehmen der flaschen- oder tubenförmigen Behälter aus dem Wasserbad ein Nachtropfen von Wasser kaum verhindert werden. In der medizinischen Diagnostik befinden sich jedoch zumeist feuchtigkeitsempfindliche Geräte im Nahbereich, etwa Ultraschallgeräte, sodass der Behälter für das Untersuchungsgel nach jeder Entnahme abgetrocknet werden müsste. Ein solcher Vorgang ist beim oftmaligen Gebrauch jedoch unpraktisch und zeitraubend.

Des Weiteren befindet sich der Flaschenkopf zumeist außerhalb des Wasserbads, sodass jener Inhalt, der sich oberhalb des Wasserspiegels befindet, nicht unmittelbar durch das Wasserbad erwärmt wird. Bei Flüssigkeiten mit vergleichsweise niedriger Viskosität spielt dieser Sachverhalt kaum eine Rolle, da durch Wärmeströmung die gesamte Flüssigkeitsmenge gleichmäßig erwärmt werden kann. In Medien höherer Viskosität, etwa bei gelartigen Medien zur medizinischen Diagnostik, kann kaum Wärme mittels Wärmeströmung übertragen werden, sodass bei gut gefüllten Behältern ausgerechnet jene Menge des Gels, die zum Einsatz kommt, nämlich jene im Öffnungsbereich nahe der Austrittsöffnung der Flasche bzw. der Tube, nicht erwärmt ist. Mit fortschreitender Leerung des Behälters sammelt sich das gelartige Medium wiederum am Boden des Behälters an, sodass der Behälter vor Gebrauch geschüttelt werden muss.

Schließlich stellt sich bei der Verwendung von Wasserbädern zur Erwärmung von Behältern in der medizinischen Diagnostik auch ein hygienisches Problem, da sich im erwärmten Wasserbad Bakterien rasch vermehren. Das Wasserbad muss daher hinreichend oft gewechselt und der Flaschenwärmer gründlich gereinigt werden.

Weitere Vorrichtungen zur Erwärmung gelartiger Medien in flaschen- oder tubenförmigen Behältern wurden in den Druckschriften US 6 575 906 B1, JP 2007 195881 A, KR 100 891 873 B1, CN 201 208 265 Y und CN 101 718 465 A beschrieben.

Es ist daher das Ziel der Erfindung eine Wärmevorrichtung für flaschen- oder tubenförmige Behälter gelartiger Medien zur medizinischen Diagnostik bereit zu stellen, die diese Nachteile vermeidet und einerseits hohen Hygieneansprüchen bei Vermeidung von Feuchtigkeit genügt und andererseits eine einfache und rasche Arbeit mit den entsprechend erwärmten Behältern erlaubt, wobei stets jene Menge des gelartigen Mediums, das zur Anwendung gelangt, zuverlässig erwärmt wird und kein Trocknen oder Schütteln des Behälters mehr notwendig ist.

Diese Ziele werden durch die Merkmale von Anspruch 1 erreicht. Anspruch 1 bezieht sich auf eine Wärmevorrichtung für flaschen- oder tubenförmige Behälter gelartiger Medien zur medizinischen Diagnostik, bei dem ein beidseits geöffnetes hülsenförmiges Gehäuse mit zwei Öffnungen und einer zylindermantelförmigen, elektrisch beheizten Innenfläche vorgesehen ist, in dessen erste Öffnung der flaschen- oder tubenförmige Behälter einschiebbar ist, bei der erfindungsgemäß vorgeschlagen wird, dass das Gehäuse mit einer, am Gehäuse lösbar befestigten Aufnahme für den flaschen- oder tubenförmigen Behälter versehen ist, wobei die Aufnahme innerhalb des, von der Innenfläche umgrenzten Bereiches angeordnet ist und einen, gegenüber der zweiten Öffnung des Gehäuses dicht ausgeführten Bodenbereich aufweist. Die erfindungsgemäße Wärmevorrichtung eignet sich somit für die Überkopflagerung des flaschen- oder tubenförmigen Behälters. Der Behälter wird dabei durch die oben liegende, erste Öffnung des Gehäuses in Richtung der darunter liegenden, zweiten Öffnung des Gehäuses eingeschoben, wobei die Austrittsöffnung des, in der Aufnahme gelagerten Behälters nach unten orientiert ist. Flaschen- oder tubenförmige Behälter für gelartige Medien zur medizinischen Diagnostik weisen zumeist eine düsenartig ausgeführte Austrittsöffnung aus, sodass das gelartige Medium nur durch Druckausübung ausgebracht werden kann. In der Regel fließt daher auch bei Überkopflagerung und unverschlossener Austrittsöffnung kein gelartiges Medium aus dem Behälter aus. Es ist aber nicht ausgeschlossen, dass ein Nachtropfen des gelartigen Mediums zu Verunreinigungen führen kann. Da die Aufnahme erfindungsgemäß einen dicht ausgeführten Bodenbereich aufweist, wird allfällig austropfendes Gel aufgefangen. Aufgrund der lösbaren Befestigung der Aufnahme am Gehäuse kann die Aufnahme jedoch leicht entnommen und gereinigt werden. Die lösbare Befestigung der Aufnahme hat aber auch den Zweck, verschiedene Aufnahmen für dasselbe Gehäuse verwenden zu können, die jeweils an unterschiedliche Behälter angepasst sind, da sich Behälter unterschiedlicher Hersteller in Form und Größe unterscheiden können. Die Überkopflagerung des Behälters stellt überdies sicher, dass sich stets gelartiges Medium im Öffnungsbereich des Behälters ansammelt, das außerdem zuverlässig erwärmt wird, da die Aufnahme innerhalb des, von der Innenfläche umgrenzten Bereiches angeordnet ist. Die elektrische Beheizung der Innenfläche lässt auf die Verwendung eines Wasserbades oder dergleichen verzichten, sodass sich keine Risiken aufgrund von Feuchtigkeit ergeben.

Eine einfache Ausführung der lösbar befestigten Aufnahme besteht darin, die Aufnahme als Bodenteil einer taschenförmig ausgebildeten Halterung auszuführen, wobei die Halterung in das hülsenförmige Gehäuse einschiebbar ist. Hierfür kann vorgesehen sein, dass die Halterung eine Mantelfläche aufweist, die zumindest abschnittsweise formschlüssig zur Innenfläche des hülsenförmigen Gehäuses ausgeführt ist und an ihrem, dem Bodenteil gegenüberliegenden Ende einen flansch- oder stulpartigen Kragen als Anschlag für das hülsenförmige Gehäuse aufweist. Die Halterung kann somit durch die erste, oben liegende Öffnung des Gehäuses eingeschoben werden, bis der flansch- oder stulpartige Kragen in Anlage mit dem Gehäuse gerät und die Halterung im Gehäuse fixiert. Der Bodenteil mit der Aufnahme kommt in dieser Position in Richtung der unten liegenden zweiten Öffnung zu liegen, vorzugsweise im Nahebereich der zweiten Öffnung. Die axiale Erstreckung der Halterung wird dabei so ausgeführt sein, dass der eingeschobene Behälter nicht gänzlich innerhalb des Gehäuses aufgenommen wird, sondern mit seinem Flaschenboden zum Teil frei liegend verbleibt, um ihn leichter entnehmen zu können. Auf die zweckdienliche Erwärmung des gelartigen Mediums hat dieser Umstand keine Auswirkung, da sich jener Teil des Gels, der tatsächlich zur Anwendung gelangt, im Kopfbereich des Behälters befindet, der jedoch innerhalb des Gehäuses liegt und somit erwärmt wird.

Hinsichtlich der Beheizung wird vorgeschlagen, dass im Gehäuse eine zylindermantelförmige Heizmanschette angeordnet ist, die die Innenfläche zumindest teilweise umschließt. Eine solche Ausführung ist baulich einfach auszuführen und in der Praxis mit einer vergleichsweise niedrigen elektrischen Leistungsaufnahme verbunden.

Zur baulichen Ausführung des Gehäuses wird insbesondere vorgeschlagen, dass das hülsenförmige Gehäuse aus einer äußeren und einer inneren Hülse gebildet wird, die exzentrisch zueinander angeordnet sind. Die Heizmanschette ist hierbei an der Außenfläche der inneren Hülse angeordnet. Die exzentrische Anordnung schafft im hülsenförmigen Gehäuse Raum zur Unterbringung elektrischer Komponenten wie Spannungsversorgung, Spannungsregler, Temperaturfühler und Thermostat. Es wäre zwar möglich, diesen Raum durch einseitigen Verschluss des Gehäuses zu schaffen, sodass er nicht mehr hülsenförmig sondern etwa becherförmig ausgeführt ist, eine solche Ausführung erschwert jedoch die Reinigung der erfindungsgemäßen Vorrichtung. Die exzentrische Anordnung der beiden Hülsen ermöglicht eine beidseitig offene Ausführung des hülsenförmigen Gehäuses und somit eine einfache Reinigung. Des Weiteren kann der Außendurchmesser der äußeren Hülse nur geringfügig größer als der Innendurchmesser gewählt werden, sodass schlanke Ausführungen mit geringerem Gewicht und geringeren Kosten möglich sind.

Die Erfindung wird im Folgenden anhand eines Ausführungsbeispiels mithilfe der beiliegenden Zeichnungen näher erläutert. Es zeigen hierbei die
Fig. 1 eine Vorderansicht einer Ausführungsform der erfindungsgemäßen Wärmevorrichtung,
Fig. 2 eine Schnittansicht A-A der Ausführungsform von Fig. 1,
Fig. 3 eine Schnittansicht B-B der Ausführungsform von Fig. 1,
Fig. 4 eine perspektivische Ansicht der Ausführungsform von

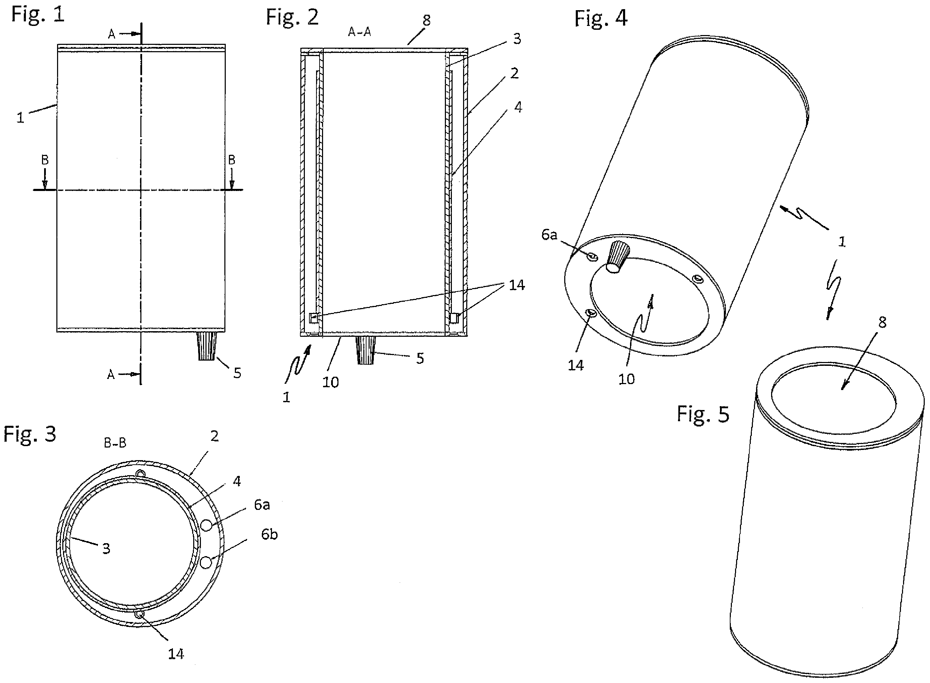

Fig. 1 von unten gesehen,
Fig. 5 eine perspektivische Ansicht der Ausführungsform von
Fig. 1 von oben gesehen,

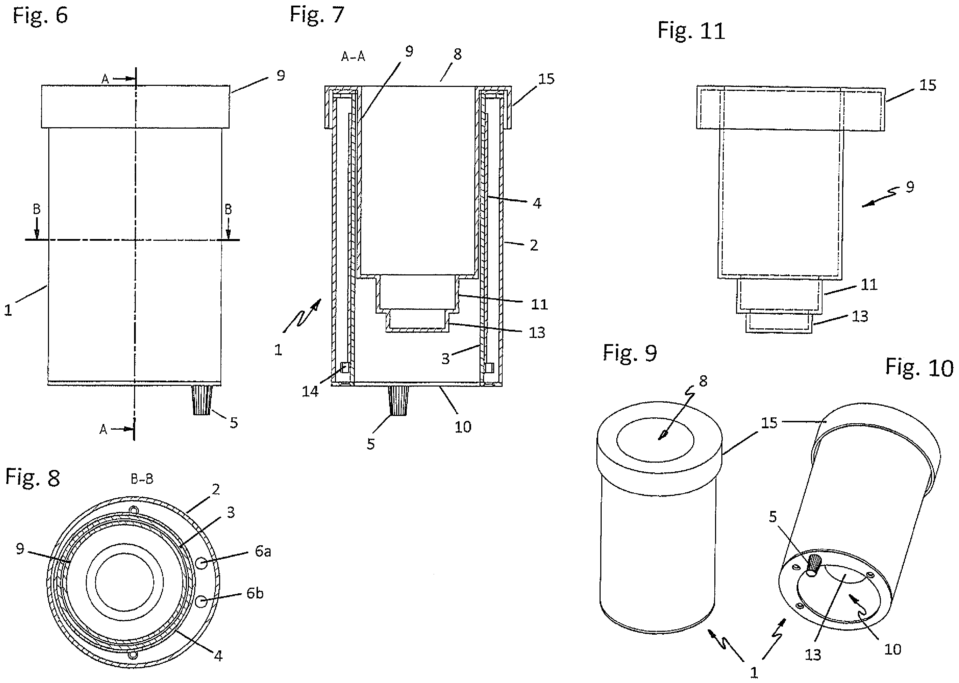

Fig. 6 eine Vorderansicht der Ausführungsform von Fig. 1 mit eingeschobener Halterung,
Fig. 7 eine Schnittansicht A-A der Ausführungsform von Fig. 6,
Fig. 8 eine Schnittansicht B-B der Ausführungsform von Fig. 6,
Fig. 9 eine perspektivische Ansicht der Ausführungsform von
Fig. 6 von oben gesehen,
Fig. 10 eine perspektivische Ansicht der Ausführungsform von
Fig. 6 von unten gesehen,
Fig. 11 eine Seitenansicht der Halterung,

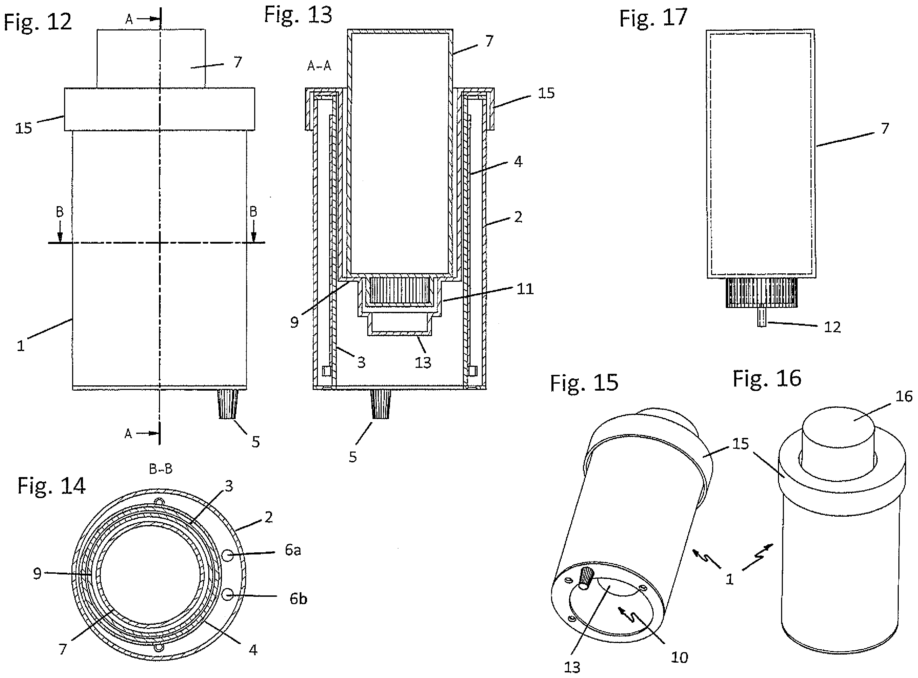

Fig. 12 eine Vorderansicht der Ausführungsform von Fig. 1 mit eingeschobener Halterung und eingeschobenem Behälter,
Fig. 13 eine Schnittansicht A-A der Ausführungsform von Fig. 12,
Fig. 14 eine Schnittansicht B-B er Ausführungsform von Fig. 12,
Fig. 15 eine perspektivische Ansicht der Ausführungsform von
Fig. 12 von unten gesehen,
Fig. 16 eine perspektivische Ansicht der Ausführungsform von
Fig. 12 von oben gesehen, und die
Fig. 17 eine Seitenansicht einer Ausführungsform eines Behälters.

Zur Erläuterung des hülsenförmigen Gehäuses 1 der erfindungsgemäßen Wärmevorrichtung wird zunächst auf die Fig. 1 bis 5 Bezug genommen. Das hülsenförmige Gehäuse 1 wird aus einer äußeren Hülse 2 und einer inneren Hülse 3 gebildet, die exzentrisch zueinander angeordnet sind, wie etwa der Fig. 3 und der Fig. 5 entnommen werden kann. Eine elektrisch betriebene Heizmanschette 4 ist hierbei an der Außenfläche der inneren Hülse 3 angeordnet und liegt dicht an ihr an, um eine optimale Wärmeübertragung von der Heizmanschette 4 auf die innere Hülse 3 zu gewährleisten. Die exzentrische Anordnung schafft im hülsenförmigen Gehäuse 1 Raum zur Unterbringung elektrischer Komponenten wie Spannungsversorgung, Spannungsregler, Temperaturfühler und Thermostat, von denen in Fig. 1, Fig. 2 und Fig. 4 lediglich der Regler 5 ersichtlich ist. In der Fig. 3 sind jedoch eine erste Bohrung 6a für einen Klinkenstecker zur Stromversorgung, sowie eine zweite Bohrung 6b für den Regler 5 ersichtlich. Die exzentrische Anordnung der beiden Hülsen ermöglicht auch eine beidseitig offene Ausführung des hülsenförmigen Gehäuses 1 und somit eine einfache Reinigung, sowie geringen Materialeinsatz zur Fertigung. Die innere Hülse 3 ist ferner mit der äußeren Hülse 2 über kreisringförmige Ober- und Unterteile fest verbunden, etwa verschweißt oder verschraubt, um insbesondere eine flüssigkeitsdichte Anordnung der elektrischen Komponenten sicher zu stellen. Das hülsenförmige Gehäuse 1 kann etwa in einen stationären Hülsenhalter eingesteckt werden, in dem es formschlüssig oder auch magnetisch gehalten ist. Das hülsenförmige Gehäuse 1 wird dabei vorzugsweise in einen Hülsenhalter mit geschlossenem Boden eingesteckt, wodurch Wärmeverluste nach unten vermieden werden. Der Hülsenhalter positioniert das Gehäuse 1 des Weiteren vorzugsweise in einer Schräglage von etwa 45°, was einerseits eine größere Kontaktfläche des Behälters 7 zur Wärmevorrichtung ermöglicht, und andererseits ein Nachtropfen des gelartigen Mediums reduziert. Des Weiteren sind in den Fig. 2 bis 4 Verschraubungen 14 der inneren Hülse 3 mit der äußeren Hülse 2 ersichtlich.

Ausführungen für die Heizmanschette 4 sind hinlänglich bekannt, sie kann etwa als Heizfolie ausgeführt sein, die auf die innere Hülse 3 aufgeklebt wird. Die zylindermantelförmige Heizmanschette 4 ist ferner über einen Klinkenstecker mit einer Stromversorgung verbunden. An der Außenfläche der inneren Hülse 3 ist ferner ein Temperaturfühler befestigt, der mit einer Steuerungseinheit verbunden ist (in den Fig. 1 bis 17 nicht ersichtlich). Die Steuerungseinheit entspricht dem Stand der Technik und umfasst im Wesentlichen einen Thermostat, der je nach gemessener Temperatur an der Außenfläche der inneren Hülse 3 die Stromzufuhr unterbricht oder herstellt. Der Sollwert der Temperatur kann über den Regler 5 vorgegeben werden, etwa in einem Temperaturbereich, der der menschlichen Körpertemperatur etwa entspricht.

Durch eine erste Öffnung 8 ist eine Halterung 9 in Richtung der zweiten Öffnung 10 des hülsenförmigen Gehäuses 1 einschiebbar. Die Halterung 9 ist etwa aus einem Kunststoff gefertigt und ist taschenförmig ausgeführt, wobei der Bodenteil der Halterung 9 eine Aufnahme 11 für den flaschen- oder tubenförmigen Behälter 7, insbesondere für dessen Öffnungsbereich, in dem sich die Austrittsöffnung 12 befindet, bildet (siehe insbesondere Fig. 7 und Fig. 11). Die Aufnahme 11 weist einen, gegenüber der zweiten Öffnung 10 des Gehäuses 1 dicht ausgeführten Bodenbereich 13 auf der als Tropfschutz für den eingelegten Behälter 7 dient. Die Aufnahme 11 ist in ihrer Formgebung an den jeweiligen Behälter 7 angepasst und nimmt ihn vorzugsweise formschlüssig auf.

Die Mantelfläche der Halterung 9 ist vorzugsweise zumindest abschnittsweise formschlüssig zur Innenfläche des hülsenförmigen Gehäuses 1 ausgeführt ist und weist an ihrem, dem Bodenteil gegenüberliegenden Ende einen flansch- oder stulpartigen Kragen 15 als Anschlag für das hülsenförmige Gehäuse 1 auf. Die Halterung 9 kann somit durch die oben liegende, erste Öffnung 8 des Gehäuses 1 eingeschoben werden, bis der flansch- oder stulpartige Kragen 15 in Anlage mit dem Gehäuse 1 gerät und die Halterung 9 im Gehäuse 1 fixiert. Der Bodenteil mit der Aufnahme 11 kommt in dieser Position in Richtung der unten liegenden zweiten Öffnung 10 zu liegen, vorzugsweise im Nahebereich der zweiten Öffnung 10, wie in der Fig. 7 ersichtlich ist.

In weiterer Folge kann in die erfindungsgemäße Wärmevorrichtung ein flaschen- oder tubenförmiger Behälter 7 eingeschoben werden, wie anhand der Fig. 12 bis 17 erläutert wird. Die erfindungsgemäße Wärmevorrichtung eignet sich insbesondere für die Überkopflagerung des flaschen- oder tubenförmigen Behälters 7. Der Behälter 7 wird dabei durch die erste, oben liegende Öffnung 8 des Gehäuses 1 in Richtung der darunter liegenden, zweiten Öffnung 10 des Gehäuses 1 eingeschoben, wobei die Austrittsöffnung 12 des, in der Aufnahme 11 gelagerten Behälters 7 nach unten orientiert ist. Flaschen- oder tubenförmige Behälter 7 für gelartige Medien zur medizinischen Diagnostik weisen zumeist eine düsenartig ausgeführte Austrittsöffnung 12 aus, wie etwa anhand der Fig. 17 ersichtlich ist, sodass das gelartige Medium nur durch Druckausübung ausgebracht werden kann. In der Regel fließt daher auch bei Überkopflagerung und unverschlossener Austrittsöffnung 12 kein gelartiges Medium aus dem Behälter 7 aus. Verunreinigungen aufgrund eines allfälligen Nachtropfens des gelartigen Mediums werden mithilfe des dicht ausgeführten Bodenbereiches 13 der Aufnahme 11 unterbunden. Aufgrund der lösbaren Befestigung der Halterung 9 und somit der Aufnahme 11 am Gehäuse 1 kann die Aufnahme 11 jedoch leicht entnommen und dessen Bodenbereich 13 gereinigt werden. Des Weiteren können aufgrund der lösbaren Befestigung der Halterung 9 und seiner Aufnahme 11 auch verschiedene Aufnahmen 11 für dasselbe Gehäuse 1 verwendet werden, die jeweils an unterschiedliche Behälter 7 angepasst sind. Die Überkopflagerung des Behälters 7 stellt überdies sicher, dass sich stets gelartiges Medium im Öffnungsbereich des Behälters 7 ansammelt, das außerdem zuverlässig erwärmt wird, da die Aufnahme 11 innerhalb des, von der Innenfläche der inneren Hülse 3 umgrenzten Bereiches angeordnet ist. Die elektrische Beheizung der Innenfläche lässt auf die Verwendung eines Wasserbades oder dergleichen verzichten, sodass sich keine Risiken aufgrund von Feuchtigkeit ergeben.

Somit erfüllt die erfindungsgemäße Wärmevorrichtung für flaschen- oder tubenförmige Behälter 7 gelartiger Medien zur medizinischen Diagnostik hohen Hygieneansprüchen bei Vermeidung von Feuchtigkeit, und erlaubt eine einfache und rasche Arbeit mit den entsprechend erwärmten Behältern 7, wobei stets jene Menge des gelartigen Mediums, das zur Anwendung gelangt, zuverlässig erwärmt wird und kein Trocknen oder Schütteln des Behälters 7 mehr notwendig ist.

## Patentansprüche

1. Wärmevorrichtung für flaschen- oder tubenförmige Behälter (7) gelartiger Medien zur medizinischen Diagnostik, bei dem ein beidseits geöffnetes hülsenförmiges Gehäuse (1) mit zwei Öffnungen (8, 10) und einer zylindermantelförmigen, elektrisch beheizten Innenfläche vorgesehen ist, in dessen erste Öffnung (8) der flaschen- oder tubenförmige Behälter (7) einschiebbar ist, **dadurch gekennzeichnet, dass** das Gehäuse (1) mit einer, am Gehäuse (1) lösbar befestigten Aufnahme (11) für den flaschen- oder tubenförmigen Behälter (7) versehen ist, wobei die Aufnahme (11) innerhalb des, von der Innenfläche umgrenzten Bereiches angeordnet ist und einen, gegenüber der zweiten Öffnung (10) des Gehäuses (1) dicht ausgeführten Bodenbereich (13) aufweist.

2. Wärmevorrichtung nach Anspruch 1, **dadurch gekennzeichnet, dass** die Aufnahme (11) als Bodenteil einer taschenförmig ausgebildeten Halterung (9) ausgeführt ist, wobei die Halterung (9) in das hülsenförmige Gehäuse (1) einschiebbar ist.

3. Wärmevorrichtung nach Anspruch 2, **dadurch gekennzeichnet, dass** die Halterung (9) eine Mantelfläche aufweist, die zumindest abschnittsweise formschlüssig zur Innenfläche des hülsenförmigen Gehäuses (1) ausgeführt ist und an ihrem, dem Bodenteil gegenüberliegenden Ende einen flansch- oder stulpartigen Kragen (15) als Anschlag für das hülsenförmige Gehäuse (1) aufweist.

4. Wärmevorrichtung nach einem der Ansprüche 1 bis 3, **dadurch gekennzeichnet, dass** im Gehäuse (1) eine zylindermantelförmige Heizmanschette (4) angeordnet ist, die die Innenfläche des hülsenförmigen Gehäuses (1) zumindest teilweise umschließt.

5. Wärmevorrichtung nach einem der Ansprüche 1 bis 4, **dadurch gekennzeichnet, dass** das hülsenförmige Gehäuse (1) aus einer äußeren Hülse (2) und einer inneren Hülse (3) gebildet wird, die exzentrisch zueinander angeordnet sind.

6. Wärmevorrichtung nach Anspruch 4 und 5, **dadurch gekennzeichnet, dass** die Heizmanschette (4) an der Außenfläche der inneren Hülse (3) angeordnet ist.

## Claims

1. A heating apparatus for bottle-shaped or tubular containers (7) for gel-like media for medical diagnostics, wherein a sleeve-like housing (1) which is open on both sides is provided with two openings (8, 10) and an electrically heated inner surface in the form of a cylindrical casing, into the first opening (8) of which the bottle-shaped or tubular container (7) can be inserted, **characterized in that** the housing (1) is provided with a receiver (11) releasably fastened to the housing (1) for the bottle-shaped or tubular container (7), wherein the receiver (11) is arranged within the region bounded by the inner surface and comprises a base region (13) which is sealed relative to the second opening (10) of the housing.

2. A heating apparatus according to claim 1, **characterized in that** the receiver (11) is formed as a base part of a retainer (9) formed in the manner of a pocket, wherein the retainer (9) can be inserted into the sleeve-like housing (1).

3. A heating apparatus according to claim 2, **characterized in that** the retainer (9) comprises a jacket surface which is formed at least in sections in a form-fitting manner in relation to the inner surface of the sleeve-like housing (1) and comprises, at its end opposite the base part, a flange-like or cuff-like collar (15) as a limit stop for the sleeve-like housing (1).

4. A heating apparatus according to one of the claims 1 to 3, **characterized in that** a heating sleeve (4) shaped in the manner of a cylinder jacket is arranged in the housing, said sleeve at least partly enclosing the inner surface of the sleeve-like housing (1).

5. A heating apparatus according to one of the claims 1 to 4, **characterized in that** the sleeve-like housing (1) is formed by an outer sleeve (2) and an inner sleeve (3), which are arranged eccentrically with respect to each other.

6. A heating apparatus according to claim 4 or 5, **characterized in that** the heating sleeve (4) is arranged on the outer surface of the inner sleeve (3).

## Revendications

1. Dispositif de chauffage pour des récipients (7) en forme de bouteille ou de tube contenant des substances sous forme de gel pour le diagnostic médical, dans lequel est prévu un boîtier (1) en forme de manchon ouvert aux deux extrémités avec deux ouvertures (8, 10) et une surface intérieure en forme d'enveloppe cylindrique chauffée électriquement, dans la première ouverture (8) duquel le récipient (7) en forme de bouteille ou de tube peut être inséré, **caractérisé en ce que** le boîtier (1) est muni d'un réceptacle (11) fixé de façon amovible sur le boîtier (1) pour le récipient (7) en forme de bouteille ou de tube, lequel réceptacle (11) est disposé à l'intérieur de la zone délimitée par la surface intérieure et présente une zone de fond (13) hermétique par rapport à la deuxième ouverture (10) du boîtier (1).

2. Dispositif de chauffage selon la revendication 1, **caractérisé en ce que** le réceptacle (11) est réalisé comme la partie de fond d'une monture (9) en forme de poche, laquelle monture (9) peut être insérée dans la boîtier (1) en forme de manchon.

3. Dispositif de chauffage selon la revendication 2, **caractérisé en ce que** la monture (9) présente une surface d'enveloppe qui est conçue au moins par zones en engagement positif avec la surface intérieure du boîtier (1) en forme de manchon et qui présente à son extrémité opposée à la partie de fond un collet (15) en forme de bride ou d'évasement formant une butée pour le boîtier (1) en forme de douille.

4. Dispositif de chauffage selon l'une des revendications 1 à 3, **caractérisé en ce qu'**est disposée dans le boîtier (1) une manchette chauffante (4) en forme d'enveloppe cylindrique qui entoure au moins partiellement la surface intérieure du boîtier (1) en forme de manchon.

5. Dispositif de chauffage selon l'une des revendications 1 à 4, **caractérisé en ce que** le boîtier (1) en forme de manchon est formé d'un manchon extérieur (2) et d'un manchon intérieur (3) disposés de façon excentrique l'un par rapport à l'autre.

6. Dispositif de chauffage selon la revendication 4 et 5, **caractérisé en ce que** la manchette chauffante (4) est disposée sur la surface extérieure du manchon intérieur (3).
